# EUROPEAN PATENT APPLICATION

(11) **EP 4 268 871 A1**
(43) Date of publication of application: **01.11.2023**
(21) Application number: 22739553.0
(22) Date of filing: 04.01.2022
(51) Int. Cl.: A61M 16/00, A61M 16/06, A61B 5/00, A61B 5/08, G16H 20/40, G16H 40/67

(54) **3G SENSOR MASK AND CONTINUOUS POSITIVE AIRWAY PRESSURE SYSTEM USING SAME**

(30) Priority: 12.01.2021 KR 20210004259
(71) Applicant: Mek Co., Ltd., Paju-si, Gyeonggi-do 10911 (KR)
(72) Inventor: KIM, Jong Cheol, Goyang-si, Gyeonggi-do 10395 (KR)
(74) Representative: Weickmann & Weickmann PartmbB
(86) International application number: PCT/KR2022/000102
(87) International publication number: WO 2022/154355

(57) **Abstract**

The present invention relates to a 3G sensor mask and a continuous positive airway pressure system using same, the mask sensing the sleep position of a patient during sleep to immediately correct pressure through a blower according to a changed sleep position if the sleep position has changed, and the system of the present invention comprises: a 3G sensor mask worn on the face of a patient to sense the sleep position of the patient during sleep; and a positive airway pressure machine, which is connected to the 3G sensor mask in a wired/wireless manner to receive sleep position information about the patient sensed through the 3G sensor mask, detects the respiratory obstruction pressure necessary for an airway opening in response to a received sleep position change of the patient, and immediately corrects treatment pressure on the basis of the detected respiratory obstruction pressure for each sleep position.

## Description

### Technical Field

The present invention relates to a continuous positive airway pressure device, and more specifically, to a pressure adjusting positive airway pressure system which instantaneously corrects the therapeutic positive pressure in accordance with a patient's position changes, and even if the patient's sleep position changes frequently, immediately controls the therapeutic pressure to a therapeutic pressure corresponding to the changed sleep position, thereby allowing the patient to maintain sleep without discomfort in breathing.

### Background Art

In general, patients suffering from snoring or symptoms such as sleep apnea can improve the quality of life through treatment using a continuous positive airway pressure (CPAP) device.

During a patient's sleep, in a case in which the respiratory volume decreases due to occurrence of apnea by the closure of the airway or the inhalation or exhalation flow rate decreases due to an increase in respiratory airway resistance, the continuous positive airway pressure device prevents the closure of the airway by continuously maintaining positive pressure (pressure higher than atmospheric pressure) by blowing in air of a predetermined pressure through a mask.

Such continuous positive airway pressure devices are divided into a fixed positive airway pressure device and an automatic positive airway pressure device. The fixed positive airway pressure device, which always blows air of a fixed pressure, has a disadvantage of not being able to actively correct various conditions of a patient, compared to the automatic positive airway pressure device. The automatic positive airway pressure device has an advantage of actively maintaining the patient's respiratory volume through pressure adjustment by a blower in accordance with the patient's condition (apnea, hypoventilation, snoring, etc.). However, the automatic positive airway pressure device takes from at least tens of seconds up to several minutes to respond to the patient's condition change as it gradually adjusts the pressure through several to tens of breaths. So, if the patient changes the patient's position during sleep, it is difficult to adjust the pressure immediately.

For example, the process of a patient changing the position during sleep occurs within a few seconds, but the process of changing the therapeutic pressure to the appropriate value takes more than several tens of seconds due to a slow response of the positive airway pressure device. Therefore, even if the patient changes the position and needs a relatively higher airway opening pressure than before, the low airway opening pressure is supplied due to the slow response of the positive airway pressure device, so the patient may enter hypoventilation or apnea. Alternatively, during the pressure change from high pressure to low pressure, airway opening pressure higher than the patient's position may be supplied to the patient, so the patient may feel discomfort in breathing and wake up from sleep.

### Disclosure

### Technical Problem

Accordingly, the present invention has been made in view of the above-mentioned problems occurring in the related art, and it is an object of the present invention to provide a continuous positive airway pressure system, which in a case that a patient's sleep position changes during sleep, detects airway closure pressure changing according to the changed sleep position, and immediately corrects the therapeutic pressure through a blower based on the detected airway closure pressure, thereby allowing the patient to maintain sleep without discomfort in breathing during sleep.

It is another object of the present invention is to provide a 3G sensor mask, which is mounted on the patient's face to assist the patient's breathing volume by supplying air of a predetermined pressure to the throat, and senses the patient's sleep position and transmits information on the sensed sleep position to a positive pressure device through wired or wireless means, thereby reflecting the patient's sleep position in real time.

It is a further object of the present invention is to provide a continuous positive airway pressure system, which in a case that the patient takes a waking position, recognizes a non-sleep state through the 3G sensor mask and switches from a sleep treatment mode to a non-sleep treatment mode, thereby enabling active treatment even for the non-sleep state that the patient awakes from sleep.

### Technical Solution

To accomplish the above-mentioned objects, according to the present invention, there is provided a continuous positive pressure system using a 3G sensor mask, including: a 3G sensor mask which is mounted on a patient's face to sense the patient's sleep position during sleep; and a positive pressure device which is connected wirelessly or non-wirelessly to the 3G sensor mask and receives sleep position information of the patient sensed through the 3G sensor mask, detects pressure necessary for airway opening and airway closure pressure in response to changes in the received sleep position of the patient, and immediately corrects the pressure through a blower based on the detected airway closure pressure and corresponding position information.

Here, the positive pressure device collects the sleep position information of the patient sensed from the 3G sensor mask through sleep monitoring and airway closure pressure information of the patient during sleep, matches the collected data, and analyzes airway closure pressure changing according to the patient's sleep position.

In another embodiment, the positive pressure device includes: an ACPAP (Auto CPAP), which controls pressure by adjusting a blower according to the patient's airway closure pressure during sleep regardless of the patient's sleep position; and a PCPAP (Position CPAP), which collects the patient's airway closure pressure information measured through the ACPAP for sleep monitoring of the patient, and simultaneously collects the patient's sleep position information sensed through the 3G sensor mask, matches the collected data to analyze the airway closure pressure changing according to the patient's sleep position during sleep, and immediately corrects the pressure based on the airway closure pressure according to the analyzed sleep position.

In another embodiment, the positive pressure device includes: an ACPAP (Auto CPAP), which controls pressure by adjusting a blower according to the patient's airway closure pressure during sleep regardless of the patient's sleep position; and a control module, which collects the patient's airway closure pressure information measured through the ACPAP for sleep monitoring of the patient, and simultaneously collects the patient's sleep position information sensed through the 3G sensor mask, matches the collected data to analyze the airway closure pressure changing according to the patient's sleep position during sleep, and immediately corrects the pressure based on the airway closure pressure according to the analyzed sleep position.

In another aspect of the present invention, there is provided a 3G sensor mask, as a mask for a positive pressure device, including: a supply hose which is mounted on a patient's face to supply air at a predetermined pressure to the throat; a 3G sensor module which senses the patient's sleep position during sleep; and, a communication unit which transmits sensed sleep position information of the patient through the 3G sensor module to the positive pressure device wirelessly or wirelessly, thereby reflecting the patient's sleep position in real time.

Moreover, the 3G sensor mask further includes a switch module which senses a location of a basic position when sensing the patient's sleep position through the 3G sensor module and sets a reference position.

In addition, the 3G sensor module is provided to be integrated with a frame which enables the 3G sensor module to be mounted on the patient's face, or is detachably mounted on the frame to be applicable to a mask for the existing positive pressure device.

### Advantageous Effects

According to the present invention, in a case in which a patient changes the position during sleep, the continuous positive airway pressure system senses the sleep position of the patient through the 3G sensor mask and immediately provides and controls airway closure pressure (referring to pressure required for airway opening, also referred to as airway opening pressure) for the patient, thereby enabling the patient to maintain sleep without sleep disturbance. In other words, even if the patient frequently changes the sleep position, the continuous positive airway pressure system according to the present invention can an immediate control to a target value without time delay, thereby relieving the discomfort in breathing caused by the patient's position change.

Furthermore, the present invention can analyze and record the airway closure pressure changing according to the patient's sleep positions in advance through the sleep monitoring process, thereby minimizing time delay and responding immediately.

In addition, the present invention can detect the airway closure pressure when the patient is lying straight and when the patient is lying sideways, analyze the airway closure pressure by gravity, and predict the airway closure pressure for each sleep position at various angles, thereby enabling pressure correction for sleep positions of various angles with one measurement.

Furthermore, the 3G sensor mask according to the present invention can sense the user's position and transmit the user's position to the positive pressure device while the patient wears the 3G sensor mask on the face, thereby reflecting the patient's sleep position in real time. Moreover, the present invention can provide the sensor module integrated to the mask or provide the sensor module detachably mounted, thereby expanding the applicable range of the sensor module by enabling the sensor module to be attached to the existing positive pressure masks.

In addition, in a case that the patient takes a waking position, the present invention recognizes a non-sleep state through the 3G sensor mask and switches from a sleep treatment mode to a non-sleep treatment mode, thereby enabling active treatment even for the non-sleep state.

### Description of Drawings

FIG. 1 is a view illustrating a continuous positive airway pressure system according to an embodiment of the present invention.
FIG. 2 is a view illustrating the configuration of a positive pressure system according to an embodiment of the present invention.
FIG. 3 is a view illustrating the configuration of a positive pressure system according to another embodiment of the present invention.
FIG. 4 is a view illustrating the configuration of a positive pressure system according to a further embodiment of the present invention.
FIG. 5 is a configuration block diagram for depicting a function of a control unit in a positive pressure device according to an embodiment of the present invention.
FIG. 6 is a view illustrating a graph detecting airway closure pressure matched according to changes in sleep position.
FIG. 7 is a view illustrating a graph predicting the airway closure pressure for each sleep position at various angles based on a gravity direction angle.
FIG. 8 is an operation flow chart for depicting a control method of a positive pressure system according to an embodiment of the present invention.

### Best Mode

Hereinafter, embodiments of the present invention will be described in detail with reference to the accompanying drawings.

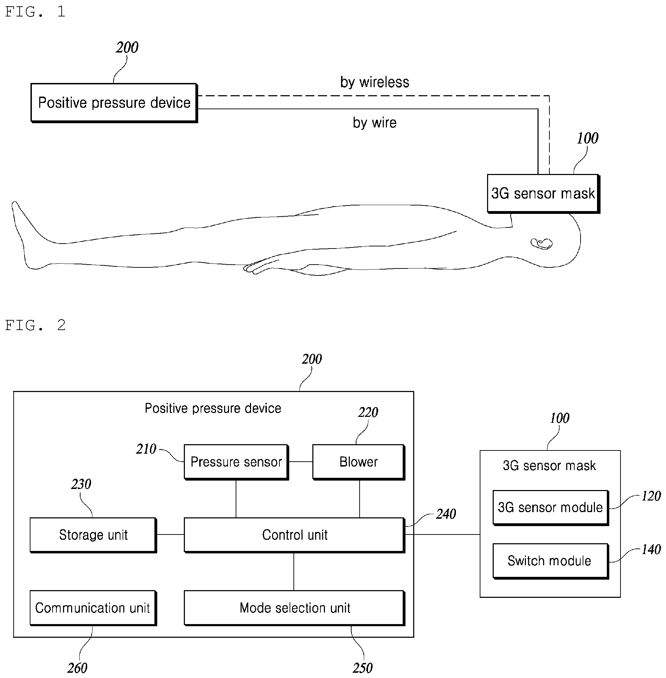

FIG. 1 is a view illustrating a continuous positive airway pressure system according to an embodiment of the present invention.

The continuous positive pressure system according to an embodiment of the present invention includes a 3G sensor mask 100, which is mounted on a patient's face, and a positive pressure device 200.

The 3G sensor mask 100, as illustrated in FIG. 1, is mounted on the patient's face to supply air of a predetermined pressure to the patient's throat, assisting the patient's respiration. Additionally, while the patient mounts the 3G sensor mask 100 on the patient's face, the 3G sensor mask 100 senses the patient's sleep position and transmits the sensed result to the positive pressure device 200. In this instance, the 3G sensor mask 100 is connected to the positive pressure device 200 by wire or wirelessly to transmit data to the positive pressure device 200.

The positive pressure device 200 receives the patient's sleep position information sensed through the 3G sensor mask 100, and detects the airway closure pressure, which may cause airway closure, in response to changes in the patient's sleep position received. Based on the detected airway closure pressure, the positive pressure device immediately corrects the pressure through the blower.

In other words, the positive pressure device 200 according to an embodiment of the present invention detects the airway closure pressure changing according to the sleep position when the patient's sleep position changes during sleep, and corrects the pressure of the blower to prevent airway closure.

To this end, the positive pressure device 200 according to an embodiment of the present invention must perform a sleep monitoring process to diagnose the patient's sleep patterns. Generally, airway closure varies depending on the sleep position, for instance, the position of lying straight and the position of lying sideways, and also varies anatomically from person to person. Typically, the airway closure due to gravity during the position of lying straight is higher than during the position of lying sideways. For the above reason, in the embodiment of the present invention, the patient's sleep position patterns and airway closure pressure patterns during sleep are collected as sleep diagnostic data through sleep monitoring, and the airway closure pressure according to the patient's sleep position is predetermined in advance. Subsequently, when the patient's sleep position changes, the present invention immediately adjusts pressure by extracting the airway closure pressure corresponding to the sleep position measured by the 3G sensor mask 100.

Hereinafter, the configuration of the system of the present invention will be described with reference to various embodiments.

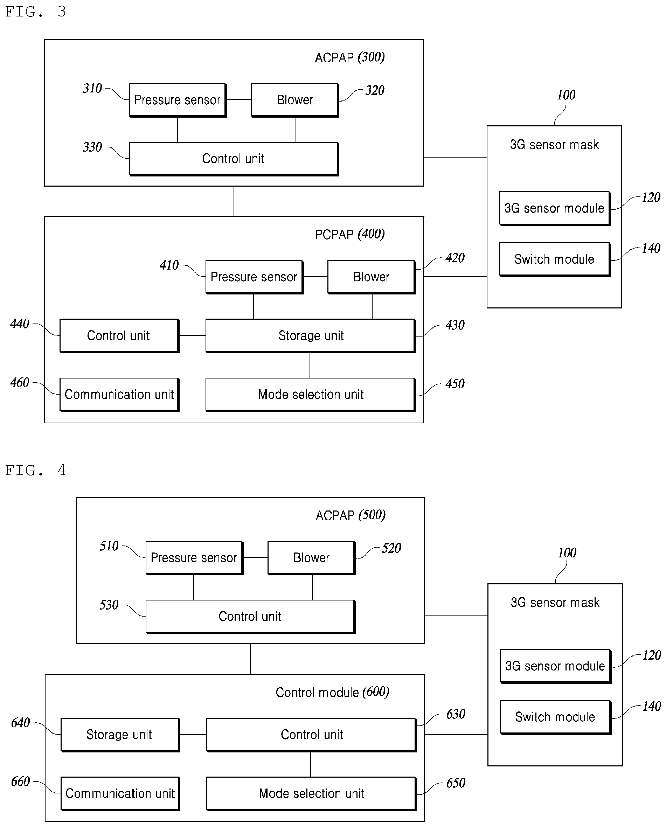

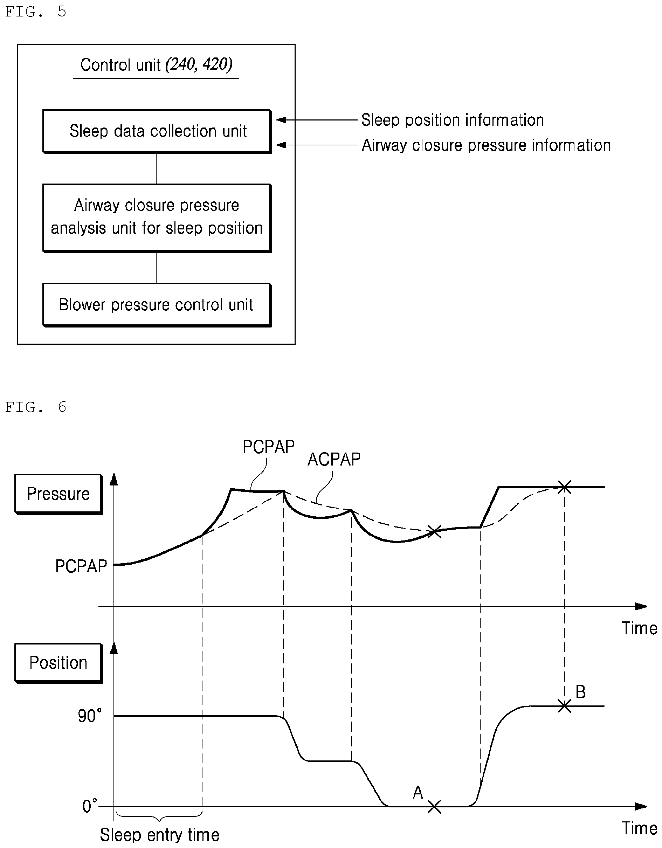

FIG. 2 is a view illustrating the configuration of a positive pressure system according to an embodiment of the present invention, FIG. 3 is a view illustrating the configuration of a positive pressure system according to another embodiment of the present invention, FIG. 4 is a view illustrating the configuration of a positive pressure system according to a further embodiment of the present invention, and FIG. 5 is a configuration block diagram for depicting a function of a control unit in a positive pressure device according to an embodiment of the present invention.

FIG. 2 illustrates a system realized by a 3G sensor mask 100 and a single positive pressure device 200, and FIG. 3 illustrates a system realized by a 3G sensor mask 100 and two positive pressure devices 300 and 400, namely, an auto continuous positive airway pressure (ACPAP) 300 and a position continuous positive airway pressure (PCPAP) 400. FIG. 4 illustrates a system in which only a control module 600 is added to the ACPAP 500 for control based on sleep positions.

Here, the ACPAP refers to the existing automatic positive pressure device, and the PCPAP refers to an automatic positive pressure device which controls pressure according to changes in sleep position by the embodiments of the present invention. Therefore, the present invention can be realized by directly applying or integrating the existing automatic positive pressure device, ACPAP, through various embodiments.

First, referring to FIG. 2, the 3G sensor mask 100 according to an embodiment of the present invention, as a mask for the positive pressure device, basically includes a supply hose which is mounted on the patient's face to supply air of a predetermined pressure to the throat, and also includes a 3G sensor module 120 which senses the patient's sleep position during sleep, and a switch module 140. Moreover, the 3G sensor mask may further include a communication unit (not shown) that transmits the patient's sleep position information sensed through the 3G sensor module 120 to the communication unit 260 of the positive pressure device 200 by a wired or wireless method.

The wired method is to connect by wire through a supply hose, and the wireless method is to connect using short-range wireless communications, such as RF (RADIO FREQUENCY) wireless communication, Bluetooth communication, WiFi communication, NFC (NEAR FIELD COMMUNICATION), Beacon, and the like.

The 3G sensor module 120 is a device which measures velocity, position, distance, etc., in a three-axis direction to sense the current position. For instance, the 3G sensor module 120 includes a three-axis acceleration sensor, a three-axis gyroscope sensor, a sensor using a laser, and the like. The power of the 3G sensor module 120 can be provided by a battery or rechargeable battery.

The switch module 140 is used to set a reference position when the patient's sleep position is sensed through the 3G sensor module 120. In other words, when the patient operates the switch module 140, the switch module recognizes a sensing value at the corresponding position as a basic position and sets the basic position as the reference position.

Additionally, when there is user manipulation for setting the reference position, the switch module 140 may provide guidance messages for maintenance of the basic position for a few seconds through voice, or a visual display method using a screen or a display light. Here, the basic position generally refers to the position of lying straight looking up at the sky, but is not limited thereto, and the user may set the position of lying sideways as the basic position according to the user's convenience.

By such a configuration, the 3G sensor mask 100 according to an embodiment of the present invention senses the patient's sleep position through the 3G sensor module 120 while being mounted on the patient's face, and transmits the sensed patient's sleep position information to the positive pressure device 200 to reflect changes in the patient's sleep position in real time.

Meanwhile, in an embodiment of the present invention, the 3G sensor module 120 may be integrated with a frame which is provided to be mounted on the patient's face, or may be provided in a detachable manner. In the latter case, since the 3G sensor module 120 can be used by being attached to the existing mask for the positive pressure device, the 3G sensor module can be expanded in the applicable range even to a patient who possesses the existing mask for the positive pressure device.

The positive pressure device 200, according to an embodiment of the present invention, includes a pressure sensor 210, a blower 220, a storage unit 230, a control unit 240, a mode selection unit 250, and a communication unit 260.

The pressure sensor 210 measures pressure of oxygen and air which are introduced, and the blower 220 sprays and supplies the introduced air to the inside.

The control unit 230 adjusts the pressure of the blower 220 in accordance with the patient's condition based on the air pressure measured through the pressure sensor 210. By the above configuration, the positive pressure device 200 according to an embodiment of the present invention can actively control the pressure of the air supplied to the patient's throat.

Furthermore, the control unit 240 receives the patient's sleep position information sensed from the 3G sensor mask 100 through the communication unit 260, and detects the pressure needed for airway opening, that is, the airway closure pressure (or referred to as airway opening pressure) in response to the changes in the received patient's sleep position. In addition, the control unit corrects the pressure through the blower 220 based on the detected airway closure pressure.

To detect the airway closure pressure for each sleep position, the control unit 200 performs a sleep monitoring process that diagnoses the sleep patterns of each patient. That is, the control unit monitors the patient's airway closure pressure during sleep, and at the same time, monitors the patient's sleep position using the 3G sensor mask 100.

As a result of the monitoring, as illustrated in FIG. 5, the control unit 240 collects the patient's sleep position information sensed through the 3G sensor mask 100 and the airway closure pressure information of the patient during sleep sensed through the pressure sensor 210 from the sleep data collection unit. Thereafter, an airway closure pressure analysis unit for each sleep position analyzes the airway closure pressure changing according to the patient's sleep position by matching the collected data.

In other words, the airway closure pressure analysis unit for each sleep position can analyze the airway closure pressure corresponding to the patient's sleep position by matching the patient's sleep position information sensed from the 3G sensor mask 100 and the airway closure pressure information of the patient during sleep sensed from the pressure sensor 210.

The method of analyzing the airway closure pressure includes the steps of: detecting the airway closure pressure when the patient is lying straight and the airway closure pressure when the patient is lying sideways from the collected data; determining each detected airway closure pressure as the maximum pressure and the minimum pressure according to the gravity direction angle; generating a waveform function (for instance, a sine waveform) that varies proportionally to the gravity direction angle within a value range between the maximum pressure and the minimum pressure; and predicting and extracting the airway closure pressure in accordance with various angles of the sleep position based on the generated function.

In other words, the airway closure pressure analysis is performed by detecting a first airway closure pressure when the patient is lying straight and a second airway closure pressure when the patient is lying sideways based on the collected data, determining one of the detected first and second airway closure pressures as the maximum pressure by the gravity direction angle and determining the other one as the minimum pressure by the gravity direction angle, generating a waveform function (for instance, a sine waveform) that varies proportionally to the gravity direction angle within an airway closure pressure value range between the maximum pressure and the minimum pressure, and predicting and extracting the airway closure pressure in accordance with various angles of the sleep position based on the generated function.

A blower pressure control unit 240 of the control unit immediately corrects the pressure of the blower based on the airway closure pressure detected through the airway closure pressure analysis Unit for each sleep position.

Through the configuration, the control unit 240 according to the embodiment of the present invention determines the airway closure pressure in advance based on the patient's sleep position through sleep monitoring, and when the patient's sleep position changes, extracts the airway closure pressure corresponding to the measured sleep position by the 3G sensor mask 100 from the function analyzed in the airway closure pressure analysis unit for each sleep position, and immediately adjusts the pressure of the Blower 220.

FIG. 6 represents a graph that detects the airway closure pressure matched in accordance with changes in sleep position.

In the graph, a ACPAP curve (dotted line) represents values obtained by monitoring and measuring the airway closure pressure of the patient during sleep, and the graph roughly shows that the ACPAP curve is gradually changed each time the patient's sleep position changes.

In this instance, a point A represents when the position is 0°, that is, when the patient is lying sideways, and a point B represents when the position is 90°, that is, when the patient is lying straight.

Through sleep monitoring, the positive pressure device according to one embodiment of the invention collects airway closure pressure information and sleep position information of the patient during sleep, acquires the ACPAP curve and a sleep position curve illustrated in FIG. 6, and can match the curves with each other.

Therefore, the positive pressure device can respectively detect the pressure at point A, i.e., the airway closure pressure when the patient is lying sideways, and the pressure at point B, i.e., the airway closure pressure when the patient is lying straight.

On the other hand, a PCPAP curve (solid line) represents values obtained by monitoring the result of performing the patient's sleep position control according to an embodiment of the present invention and measuring the airway closure pressure according to an embodiment of the present invention Comparing the PCPAP curve with the ACPAP curve, the two curves form almost similar waveforms, but it is shown that the PCPAP curve actively changes each time the patient's sleep position changes. In other words, the two curves show the results that an active immediate control is performed in accordance with the patient's sleep position without time delay.

In addition, during the time for the onset of sleep before falling asleep in the PCPAP curve, pressure is gradually controlled along the ACPAP curve. In general, when the patient enters sleep, the airway closure pressure gradually rises more than usual, so the present invention controls the pressure gradually like the ACPAP operation mode regardless of the sleep position. It actively controls depending on the changes in sleep position after the onset of sleep.

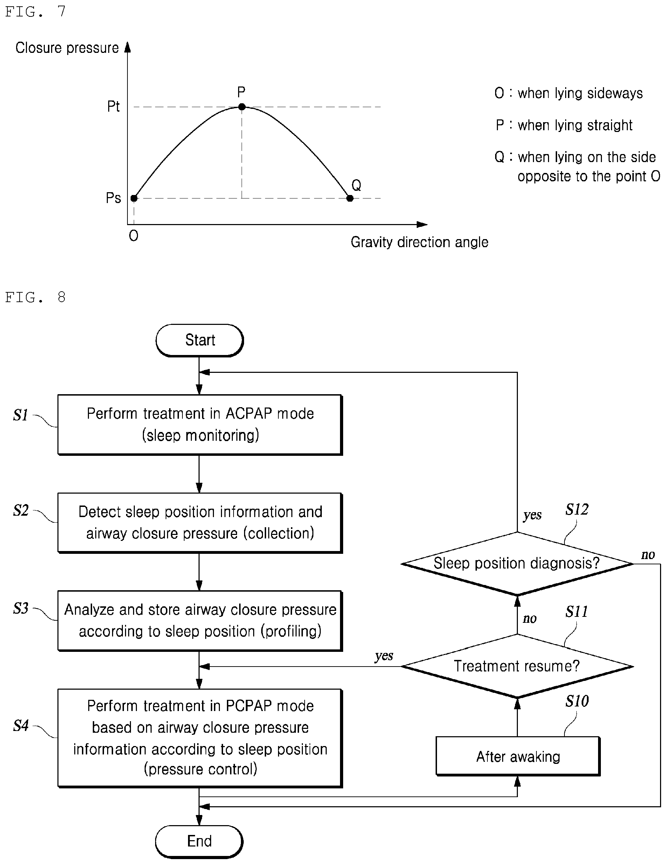

FIG. 7 is a view illustrating a graph predicting the airway closure pressure for each sleep position at various angles based on a gravity direction angle.

The airway closure pressures detected in the graph of FIG. 6, namely, the airway closure pressure (Pt) when the patient is lying straight, and the airway closure pressure (Ps) when the patient is lying sideways, are respectively set as the maximum pressure and the minimum pressure by the gravity direction angle, and the three-axis information is synthesized and expressed as the gravity direction angle. Then, the airway closure pressure proportional to the gravity direction angle can be predicted with a sine waveform.

Based on such curves, the positive pressure device according to an embodiment of the invention can predict and extract the corresponding airway closure pressure for the sleep position at various angles.

The gravity direction angle refers to an angle converted state in the gravity direction by synthesizing the information of the lying position and neck position, etc., the X, Y, and Z axes. From an anatomic perspective, the proportion of the X, Y, and Z axes may be reflected differently.

In the curve of FIG. 7, a point O means the position of lying sideways, a point P means the position of lying straight, and a point Q means the position of lying on the side opposite to the point O.

Referring back to FIG. 2, when the patient's upright position is sensed from the 3G sensor mask 100, the control unit 240 according to an embodiment of the invention recognizes it as a non-sleep state and switches the sleep treatment mode to a non-sleep treatment mode, linked with the mode selection unit 250 to control the operation.

The mode selection unit 250 divides the treatment mode into the sleep treatment mode and the non-sleep treatment mode and enables operation entry into the non-sleep treatment mode through a simple mode change (or selection). Generally, when the patient enters sleep, the airway closure pressure rises more than usual. Therefore, the non-sleep treatment mode is set to generally lower the pressure control compared to the sleep treatment mode.

Furthermore, when the patient wakes up from sleep or when the patient is in the non-sleep treatment mode and then tries to enter the sleep mode, the control unit 240 according to an embodiment of the invention determines whether to resume treatment in the pressure control method according to the sleep position. If a request for treatment resumption is input from the user, the control unit 240 controls operation in the pressure control method according to each patient's sleep position which has been profiled.

Meanwhile, the control unit 240 according to an embodiment of the invention profiles patients by remotely monitoring and adjusting pressure when performing sleep monitoring, or by storing and recording sleep diagnostic data in the storage unit 230 of the positive pressure device 200.

Next, referring to FIG. 3, another embodiment of the present invention will be described.

Another embodiment of the present invention shows an example realized by an ACPAP 300 and a PCPAP 400.

That is, the ACPAP 300 includes basic components such as a pressure sensor 310, a blower 320, and a control unit 330, and controls pressure by adjusting the blower according to the patient's airway closure pressure during sleep regardless of the sleep position. The ACPAP 300 is applied for a certain period of time (for example, for a night) to monitor the patient's sleep patterns, and after the monitoring, the PCPAP 400, which will be described later, is applied.

Such an ACPAP 300 has the function of a typical positive pressure device, so the existing automatic positive pressure device may be applied as it is.

The PCPAP 400 is provided separately from the ACPAP 300 and can be connected either wired or wirelessly to the ACPAP 300 and the 3G sensor mask 100 through the communication unit 460.

The PCPAP 400 includes the control unit 430, the storage unit 440, the mode selection unit 450, and the communication unit 460, similar to the configuration in the embodiment of FIG. 2. That is, the PCPAP 400 receives the patient's sleep position information sensed from the 3G sensor mask 100 via the communication unit 460, and the control unit 420 detects the airway closure pressure corresponding to the patient's sleep position changes transmitted through the communication unit 460, and then corrects the pressure through the blower 420 based on the detected airway closure pressure.

The PCPAP 400 collects the patient's airway closure pressure information measured through the ACPAP 300 in the control unit 420, and at the same time, collects the patient's sleep position information sensed through the 3G sensor mask 100. Thereafter, the PCPAP matches the collected data and analyzes the airway closure pressure, which varies according to the patient's sleep position during sleep. Based on the analysis results, the PCPAP 400 immediately corrects the pressure based on the airway closure pressure according to the sleep position.

A specific method for analyzing the airway closure pressure is the same as described in FIGS. 2, 5, 6, and 7. In addition, the configuration of the 3G Sensor mask 100 is the same as described in FIG. 2, so the overlapping configurations with the previously description will be omitted.

Therefore, the system according to another embodiment of the present invention performs sleep monitoring using the ACPAP 300, and collects and analyzes monitored data in the PCPAP 400. That is, the system initially performs treatment in the ACPAP mode through the ACPAP 300 for sleep monitoring, and after data analysis of the airway closure pressure according to the sleep position, profiling for each patient is performed, and then, treatment in the PCPAP mode is performed based on the airway closure pressure information according to the sleep position through the PCPAP 400. In other words, the system according to another embodiment of the invention of FIG. 3 includes the PCPAP separately from the existing automatic positive pressure device, the ACPAP. However, since the existing positive pressure device can be directly applied, the patient can receive active treatment according to the sleep position without preparing a new device, only by equipping an additional configuration.

Next, referring to FIG. 4, a further embodiment will be described.

In another embodiment of the invention, a control module 600, which performs pressure control according to sleep position, is added to the existing automatic positive pressure device, the ACPAP 500. The control module 600 includes a control unit (630), a storage unit 640, a mode selection unit 650, and a communication unit 660.

The control module 600, similar to the PCPAP 400 illustrated in FIG. 3, collects the patient's airway closure pressure information measured through the ACPAP 500 for patient's sleep monitoring, and simultaneously collects the patient's sleep position information sensed through the 3G sensor mask 100. The control module 600 matches the collected data and analyzes the airway closure pressure which varies according to the patient's sleep position during sleep. Then, based on the analysis results, the control module 600 immediately corrects the pressure according to the sleep position based on the airway closure pressure.

In this instance, the control module 600 differs from the embodiment of FIG. 3 in that the control module 600 controls the pressure by immediately adjusting the blower of the ACPAP 500.

However, since the existing positive pressure device can be directly applied to the control module according to this embodiment just like the embodiment in FIG. 3, the patient can receive active treatment according to the sleep position without preparing a new device, only by equipping an additional configuration.

FIG. 8 is an operation flow chart for depicting a control method of a positive pressure system according to an embodiment of the present invention.

First, in step S1, the control unit starts sleep monitoring by manipulation of a patient or a user (a nurse, or a doctor). During sleep monitoring, the control unit performs treatment for a certain period of time in the ACPAP mode. Here, the ACPAP mode refers to a control method not by the pressure control method according to the sleep position, but by the general automatic positive pressure device, that is, a method of controlling according to the patient's breathing condition.

Next, at the same time with the start of sleep monitoring, the 3G sensor mask 100 operates to sense the patient's sleep position during sleep.

Next, in step S2, the control unit detects and collects the patient's sleep position information sensed through the 3G sensor mask 100 and the airway closure pressure information treated in the ACPAP mode.

Next, in step S3, the control unit matches the collected data and analyzes and stores the airway closure pressure information according to the sleep position in the storage unit. The control unit can profile each patient through the storage.

Specifically, the control unit detects the airway closure pressure when the patient is lying straight and the airway closure pressure when the patient is lying sideways, and based on the detected airway closure pressure, predicts and detects the airway closure pressure according to the gravity direction angle of the sleep position.

Next, in step S4, the control unit performs treatment in the PCPAP mode according to the sleep position based on the airway closure pressure information. That is, based on the airway closure pressure according to the analyzed sleep position changes of each patient, the control unit immediately corrects (controls) the blower to supply the optimal pressure according to the sleep position.

Next, in steps S10 and S11, when the patient wakes up from sleep or when the patient is in the non-sleep treatment mode and then tries to enter the sleep mode, the control unit determines whether to resume treatment before operating in the PCPAP mode.

Generally, since the positive pressure device of the invention is operated on the premise that the patient falls asleep, this step is to confirm whether to continue treatment if the patient wakes up.

In step S11, if an operation to resume the treatment is input by the user, the step returns to the step S4, and the treatment is continuously performed in the PCPAP mode based on the airway closure pressure according to the sleep position, as before.

In step S11, if an operation not to resume the treatment is input by the user, like the next step S12, the control unit checks whether to diagnose to re-check the sleep position.

If diagnosed, the control unit returns to the step S1 and performs the routine operation.

If not diagnosed, the control unit ends.

Till now, the present invention has been described based on the configuration of the system integrated with the single positive pressure device. However, in the case of the system which includes the PCPAP or the control module added to the existing positive pressure device as illustrated in FIGS. 3 and 4, the treatment is performed through the existing positive pressure device, the ACPAP, in the sleep monitoring step (S1), and then, the data analysis and operation will be performed through the PCPAP or the control module.

The above description merely illustrates the invention as an example, and various modifications are possible within the scope not departing from the technical idea of the invention by a person having ordinary knowledge in the technical field to which the invention belongs. Therefore, the embodiments disclosed in the specification of the invention do not limit the present invention. The scope of the invention should be interpreted by the following claims, and various equivalents and modification examples that can replace them at the time of this application should be interpreted as included in the scope of the invention.

### Mode for Invention

Various embodiments have been described in the best form to carry out the present invention.

### Industrial Applicability

The invention is used in fields related to the continuous positive pressure system using the 3G sensor mask.

It is obvious to those skilled in the art that various changes and modifications can be made in the invention without departing from the concept or scope of the invention. Therefore, it is intended that the present invention includes changes and modifications provided within the scope of the attached claims and their equivalents.

### DRAWINGS

FIG. 1
   200: Positive pressure device
   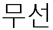 : by wireless
   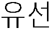 : by wire
   100: 3G sensor mask
FIG. 2
   210: Pressure sensor
   220: Blower
   230: Storage unit
   240: Control unit
   250: Mode selection unit
   260: Communication unit
   100: 3G sensor mask
   120: 3G sensor module
   140: Switch module
FIG. 3
   310: Pressure sensor
   320: Blower
   330: Control unit
   100: 3G sensor mask
   120: 3G sensor module
   140: Switch module
   410: Pressure sensor
   420: Blower
   430: Storage unit
   440: Control unit
   450: Mode selection unit
   460: Communication unit
FIG. 4
   100: 3G sensor mask
   120: 3G sensor module
   140: Switch module
   510: Pressure sensor
   520: Blower
   530: Control unit
   600: Control module
   630: Control unit
   640: Storage unit
   650: Mode selection unit
   660: Communication unit
FIG. 5
   240, 420: Control unit
   Sleep data collection unit
   Sleep position information
   Airway closure pressure information
   Airway closure pressure analysis unit for sleep position
   Blower pressure control unit
FIG. 6
   Sleep entry time
FIG. 7
   Closure pressure
   Gravity direction angle
   O: when lying sideways
   P: when lying straight
   Q: when lying on the side opposite to the point O
FIG. 8
   Start
   S1: Perform treatment in ACPAP mode (sleep monitoring)
   S2: Detect sleep position information and airway closure pressure (collection)
   S3: Analyze and store airway closure pressure according to sleep position (profiling)
   S4: Perform treatment in PCPAP mode based on airway closure pressure information according to sleep position (pressure control)
      End
   S12: Sleep position diagnosis?
   S11: Treatment resume?
   S10: After awaking

## Claims

1. A continuous positive pressure system using a 3G sensor mask, comprising:
a 3G sensor mask which is mounted on a patient's face to sense the patient's sleep position during sleep; and
a positive pressure device which is connected wirelessly or non-wirelessly to the 3G sensor mask and receives sleep position information of the patient sensed through the 3G sensor mask, detects pressure necessary for airway opening and airway closure pressure in response to changes in the received sleep position of the patient, and immediately corrects the pressure through a blower based on the detected airway closure pressure and corresponding position information.

2. The continuous positive pressure system according to claim 1, wherein the positive pressure device collects the sleep position information of the patient sensed from the 3G sensor mask through sleep monitoring and airway closure pressure information of the patient during sleep, matches the collected data, and analyzes airway closure pressure changing according to the patient's sleep position.

3. The continuous positive pressure system according to claim 1, wherein the positive pressure device includes:
an ACPAP (Auto CPAP), which controls pressure by adjusting a blower according to the patient's airway closure pressure during sleep regardless of the patient's sleep position; and
a PCPAP (Position CPAP), which collects the patient's airway closure pressure information measured through the ACPAP for sleep monitoring of the patient, and simultaneously collects the patient's sleep position information sensed through the 3G sensor mask, matches the collected data to analyze the airway closure pressure changing according to the patient's sleep position during sleep, and immediately corrects the pressure based on the airway closure pressure according to the analyzed sleep position.

4. The continuous positive pressure system according to claim 1, wherein the positive pressure device includes:
an ACPAP (Auto CPAP), which controls pressure by adjusting a blower according to the patient's airway closure pressure during sleep regardless of the patient's sleep position; and
a control module, which collects the patient's airway closure pressure information measured through the ACPAP for sleep monitoring of the patient, and simultaneously collects the patient's sleep position information sensed through the 3G sensor mask, matches the collected data to analyze the airway closure pressure changing according to the patient's sleep position during sleep, and immediately corrects the pressure based on the airway closure pressure according to the analyzed sleep position.

5. The continuous positive pressure system according to claim 1, wherein the positive pressure device detects the airway closure pressure when the patient is lying straight and the airway closure pressure when the patient is lying on the side from the collected data, sets the detected airway closure pressures as the maximum pressure and the minimum pressure by gravity direction angles, and predicts and extracts the airway closure pressure according to the sleep positions at various angles based on the function varying proportionally to the gravity direction angles within the range between the maximum pressure and the minimum pressure.

6. The continuous positive pressure system according to claim 1, wherein when the position of the patient getting up is sensed through the 3G sensor mask, the positive pressure device recognizes it as a non-sleep state, and switches the mode not into a sleep treatment mode but into a non-sleep treatment mode to enable continuous treatment.

7. The continuous positive pressure system according to claim 1, wherein, in order to set a reference position when sensing the patient's sleep position, the 3G sensor mask senses the position of the basic position and sends the basic position to the positive pressure device, and
wherein the positive pressure device sets the position received from the 3G sensor mask as the reference position, and outputs a guidance on the completion of setting and sensing operation to the user using one or more of voice, alarm, and light.

8. The continuous positive pressure system according to claim 1, wherein the positive pressure device performs remote monitoring and sleep monitoring for pressure adjustment, or performs sleep monitoring for patient profiling by storing and recording the collected data in a storage unit.

9. The continuous positive pressure system according to claim 1, wherein when the patient wakes up, the positive pressure device determines whether to resume treatment with the pressure control method according to the sleep position, and if an operation request for resuming treatment is inputted by the user, the positive pressure device performs the operation with the pressure control method according to the profiled sleep position for each patient.

10. A 3G sensor mask, as a mask for a positive pressure device, comprising:
a supply hose which is mounted on a patient's face to supply air at a predetermined pressure to the throat;
a 3G sensor module which senses the patient's sleep position during sleep; and,
a communication unit which transmits sensed sleep position information of the patient through the 3G sensor module to the positive pressure device wirelessly or wirelessly, thereby reflecting the patient's sleep position in real time.

11. The 3G sensor mask according to claim 10, further comprising:
a switch module which senses a location of a basic position when sensing the patient's sleep position through the 3G sensor module and sets a reference position.

12. The 3G sensor mask according to claim 10, wherein the 3G sensor module is provided to be integrated with a frame which enables the 3G sensor module to be mounted on the patient's face, or is detachably mounted on the frame to be applicable to a mask for the existing positive pressure device.
